# EUROPEAN PATENT SPECIFICATION

(11) **EP 4 218 625 B1**
(45) Date of publication and mention of the grant of the patent: **02.04.2025**
(21) Application number: 22154524.7
(22) Date of filing: 01.02.2022
(51) Int. Cl.: A61B 17/22

(54) **A DUAL CATHETER ARRANGEMENT AND SYSTEM FOR REPERFUSION OF AN ISCHEMIC TISSUE REGION VIA A CORONARY VESSEL**
DOPPELKATHETERANORDNUNG UND SYSTEM ZUR REPERFUSION EINES ISCHÄMISCHEN GEWEBEBEREICHS MITTELS EINES KORONARGEFÄSSES
AGENCEMENT DE CATHÉTER DOUBLE ET SYSTÈME POUR LA REPERFUSION D'UNE RÉGION DE TISSU ISCHÉMIQUE PAR L'INTERMÉDIAIRE D'UN VAISSEAU CORONAIRE

(43) Date of publication of application: 02.08.2023
(73) Proprietor: Albert-Ludwigs-Universität Freiburg, 79098 Freiburg (DE)
(72) Inventor: ZGIERSKI-JOHNSTON, Callum, 79098 Freiburg (DE); KOHL, Peter, 79098 Freiburg (DE); CHLEILAT, Enaam, 79098 Freiburg (DE)
(74) Representative: Rösler, Uwe

(56) References cited:
- EP-A2- 0 249 338
- WO-A1-2021/003133
- US-A1- 2017 189 654
- US-A1- 2021 402 158
- US-A1- 2022 000 498

## Description

### BACKGROUND OF THE INVENTION

### Field of the Invention

The invention relates to a dual catheter arrangement and a system in which the dual catheter arrangement serves as a central medical intervention tool for reperfusion of an ischemic tissue region via a coronary vessel.

### Description of the Prior Art

Myocardial ischemia occurs when blood flow to the heart muscle (myocardium) is reduced below levels required to serve tissue demand. This may be by a partial or complete blockage of a coronary artery by buildup of atherosclerotic plaques. If the atherosclerotic plaque ruptures, thrombi can form and can completely block the vessel, resulting in medical emergencies such as heart attack (myocardial infarction) or stroke.

Following myocardial ischemia caused by full or partial occlusion of a coronary artery, current treatment typically involves interventions that focus on 'reperfusion' of the ischemic tissue as quickly as possible, often followed by implantation of a stent into the vessel to prevent re-occlusion.

In this context, a catheter system is known from document US 2018/0280172 A1 which enables reperfusion of a coronary tissue region and delivering a stent for remaining in place. The catheter system comprising a proximal manifold, including an infusion port and a stent balloon inflation port, and a catheter, extending distally from the manifold and defining an infusion lumen in fluid communication with the infusion port, and an inflation lumen in fluid communication with the stent balloon inflation port. The catheter further provides a therapeutic agent port in fluid communication with a therapeutic agent lumen leading to the distal end of the catheter. Near the distal end of the catheter a stent balloon is arranged which is in fluid communication with the inflation lumen and surrounding the catheter. At the outer surface of the stent balloon a stent is attached for intravascular permanent placement.

Document US 10,952,883 B2 discloses the use of the catheter system previously described and a treatment for microvascular obstructions while avoiding reperfusion injuries. Initially the catheter including the stent will be navigated to a target location within a vessel using a guidewire first and advancing the catheter over the guidewire. Subsequently the stent balloon disposed within the stent will be inflated, thereby expanding the stent against a vessel wall at the target location and transiently occluding the vessel. Subsequently infusing an infusate into the vessel downstream of the balloon through the infusion lumen and analysing pressure and temperature for monitoring an effect of the infusion of the infusate. Finally the balloon will be deflated once a desired effect of the infusion of the infusate has been reached based on the analysis, while leaving the expanded stent in place as a safeguard against re-occlusion of the vessel.

Unfortunately reperfusion after an ischemic period can itself lead to potentially fatal arrhythmias. These arrhythmias, induced by reperfusion, are mostly considered as a necessary evil that can be treated by way of controlled defibrillation, although defibrillation can cause additional tissue damage despite its efficacy.

Some of the more advanced approaches have explored perfusion with various washing solutions before restoring blood flow to reduce ischemia-related tissue damage and/or the likelihood of arrhythmias. However, the success of these attempts has remained limited in terms of reducing arrhythmogenesis.

US 2022/0000498 A1 discloses a catheter system for removal of a thrombus from a vessel having an inner catheter with a balloon and an outer sheath with a balloon.

### SUMMARY OF THE INVENTION

It is an object of the invention to provide a catheter system that allows a reliable treatment of myocardial ischemia without the subsequent occurrence of reperfusion arrhythmias. It is a further aspect of the invention to provide a catheter system that allows an easy and reliable catheter handling and to ensure that the catheter treated myocardial tissue region is set in a state that allows a sufficient grade of blood supply to the myocardium.

The solution of the object underlying the invention is specified in Claim 1 and addresses a dual catheter arrangement. Furthermore, a system for reperfusion of an ischemic tissue region via a coronary vessel comprising the dual catheter arrangement is defined in claim 9. The features which improve upon the idea of the approach in an advantageous manner are the subject matter of the dependent claims as well as the additional description with reference to the embodiments. Methods as such do not form part of the invention.

The invention is based on the finding from animal experiments and computer simulations performed by the inventors that a two-stage reperfusion method, in which a distal region of the ischemic tissue area is re-perfused with a normal physiological infusate earlier than a proximal tissue area, can significantly reduce arrhythmogenesis. This finding is based on the understanding of reasons for the formation of ischemia-reperfusion-related arrhythmias obtained in the animal studies as follows.

In the normal state, the electrical signals generated by the heart's primary pulse generator (sinus node) are transmitted in an orderly sequence to coordinate the heart's pumping activity. If there is a blockage in a coronary artery, insufficient oxygen is supplied to the muscle cells and acidification and an increase in extracellular potassium occurs. This results in an ischemic zone in the region of the heart muscle supplied by this artery. The consequences of this are reduced electrical excitability and a slowing down or complete blockade of electrical excitation in this region of the heart muscle.

If the vascular blockage is removed using conventional reperfusion, i.e. by means of a reperfusion catheter known per se, as described before, the stenosed cardiac vascular region is permanently dilated by placement of a stent, and the ischemic myocardial region is washed with either blood or a reperfusion solution, there is subsequently increased heterogeneity of electrophysiological properties, particularly in a border zone peripherally surrounding the reperfused myocardial region, which may act as a trigger for arrhythmias before subsequent full or partial recovery of the ischemic tissue.

In addition to this mechanism it turns out, that myocardium along the reperfused coronary artery returns to electrical excitability temporally before the bulk of the ischemic region, resulting in the formation of an electrical conduction pathway through the midst of the ischemic region treated by reperfusion. This phenomenon may be named PeriVascular Excitation Tunneling, abbreviated PVET, and may lead to reentrant arrhythmias.

To effectively counteract the aforementioned PVET mechanism for the occurrence of cardiac arrhythmias, the already briefly mentioned two-stage reperfusion technique is proposed inventively, which is abbreviated as SHIELD-EP, which means "Step-wise Haemodynamic recovery of IschEmic myocardium to Lessen Deleterious effects on cardiac ElectroPhysiology".

Especially for performing SHIELD-EP an inventively designed dual catheter arrangement is proposed as follows:
A dual catheter arrangement for reperfusion of an ischemic tissue region via a coronary vessel, comprising a primary balloon catheter having a lumen for inflation and deflation of a primary balloon disposed at a distal region of the primary balloon catheter, and at least two separate lumens both extending throughout the primary balloon catheter and open at a distal end of the primary balloon catheter, and a secondary balloon catheter having a lumen for inflation and deflation of a secondary balloon disposed at a distal region of the secondary balloon catheter and an irrigation lumen opening at a distal end of the secondary balloon catheter, which is arranged slideable within one of the lumens of the primary balloon catheter to achieve a working position in which the secondary balloon catheter continues completely through the lumen of the primary balloon catheter and extends distally beyond the primary balloon catheter.

Each lumen of the primary and secondary balloon catheter provides an access point at the proximal end of the respective catheter.

Of central importance is the sliding positioning of the secondary balloon catheter within the primary balloon catheter so that the final distance between primary and secondary balloon in the working position can be individually adjusted with respect to the respective anatomical conditions.

As will be described further in detail with reference to the illustrated embodiments, to perform the SHIELD-EP method, the distal end of the primary catheter is positioned in close proximity to the location of the stenosis so that the stenosed myocardial tissue region can be dilated by means of the primary balloon. The secondary catheter protrudes through the primary catheter, with a secondary catheter region protruding beyond the first catheter to lie within the ischemic tissue region, i.e. past the stenosed or otherwise blocked proportion of the vessel. The length of the secondary catheter region protruding beyond the first catheter is adjustable with respect to the dimension of the ischemic tissue region. In the working position the secondary balloon is dilated to define two separate zones for fluid reperfusion: a proximal one between primary and secondary balloons, and a distal one distal to the secondary balloon.

After having placed the dual catheter arrangement in the working position the distal ischemic myocardial zone is reperfused by a controlled release of a liquid which preferably is a physiological perfusion solution through the irrigation lumen of the secondary balloon catheter until natural electrical excitability is restored in this distal myocardial zone. By reperfusing the distal ischemic zone in a first step only, PVET cannot give rise to uncontrolled reentrant excitation as the proximal ischemic myocardial zone is left inexcitable, thus acting as a shield-region of electrical block, conceptually similar to scars in ablation therapy.

Upon subsequent complete reperfusion, which is performed in a timely manner following recovery of the distal zone by deflating the secondary balloon and controlled release of perfusion solution through one of the lumens of the primary balloon catheter, PVET in the proximal myocardial zone may be present, but now the path length and hence the associated time-delay in electrical activation are too short to give rise to re-entrant excitation. Thus by dividing reperfusion process into two spatially and temporally separated steps, the arrhythmogenic mechanisms present upon standard reperfusion are diffused and become functionally silent. Subsequently the heart will fully recover without the occurrence of PVET-based reentrant arrhythmias.

In an alternative use of the same embodiment, the proximal perfusion zone may be irrigated with oxygenated cardioplegic solution through one of the lumens of the primary balloon catheter at the same time as perfusion of the distal zone using physiological perfusion solution applied through the irrigation lumen of the secondary catheter is conducted. This restores nutrients and removes waste products in the tissue fed by the proximal perfusion zone, while maintaining inexcitability in the tissue shield-region.

In a preferred embodiment of the dual catheter arrangement an electrode arrangement is attached at the distal end of at least at one of the primary and secondary balloon catheters, which is designed to detect recovery of electrical activity of the cardiac tissue surrounding the distal ends of the primary and secondary balloon catheter respectively.

For example, in order to determine whether natural electrical excitability is reestablished in the distal myocardial zone after a certain period of perfusion, at least one electrode is attached to the distal tip of the secondary balloon catheter, which is preferably galvanically connected to an external - and during use extracorporeal - measuring unit, to allow assessment of electrical activity in the distal myocardial zone. For the same purpose the primary balloon catheter has an electrode arrangement at its distal region of the same kind.

After completion of the first step of the SHIELD-EP method, the secondary balloon is deflated and the secondary balloon catheter may or may not remain in place while perfusion solution is delivered to the proximal and distal myocardial zone through one of the lumens of the primary balloon catheter. To more closely monitor the recovery process of electrical excitability within the proximal myocardial zone, a preferred embodiment features a further electrode arrangement proximal to the secondary balloon catheter at least in regions along its longitudinal extent. The further electrode arrangement, which preferably provides at least two, for example annular, electrodes arranged at a distance from one another along the outer circumference of the secondary primary balloon catheter, which are preferably galvanically connected to an external - and during use extracorporeal - measuring unit, designed to detect an electrical activity in the myocardium surrounding the secondary balloon catheter. For the same or a similar purpose, the primary balloon catheter preferably also has a further similar electrode arrangement along its outer circumference proximally to the balloon.

The secondary balloon catheter provides a catheter length which is greater than the length of the primary balloon catheter, so that, in the working position, the secondary balloon catheter extends distally past the primary balloon catheter by a length which normally will not exceed 10 cm. Depending on the anatomical proportions of a coronary ischemic myocardial area to be treated, it is thus possible to adjust the distance between the primary and secondary balloon variably between a few millimeters and multiple centimeters.

In addition, the secondary balloon catheter has an outer diameter that is slightly less than the inner diameter of at least one of the lumens of the primary balloon catheter into which the secondary balloon catheter is slid.

Preferably, but not necessarily, the secondary balloon catheter is completely removable from and re-insertable into the primary balloon catheter, i.e., this lumen can be used in place of the secondary catheter with any other medical instrument of appropriate dimension, for example, in the form of a guidewire, an ablation-, aspiration- or resection-catheter, to name a few.

Both the irrigation channel of the secondary balloon catheter and the at least one lumen of the primary balloon catheter that is not used by the secondary balloon catheter or other medical instrument have a connecting flange proximally for detachable, fluid-tight connection to an irrigation fluid source.

The inventive dual catheter arrangement is part of an interventional cardiology system for performing the SHIELD-EP method, which is composed of the following components:
- inflation and deflation units connected fluid-tightly with the lumens for inflation and deflation of the primary and of the secondary balloons,
- a flushing source unit connected fluid-tightly with one of the at least two lumens of the primary balloon catheter and with the irrigation lumen of the secondary balloon catheter, and
- a control unit for controlling the inflation and deflation unit and the flushing source unit in the following manner:
   a) inflating the primary balloon by the inflation and deflation unit,
   b) after placing the secondary balloon catheter in the working position, inflating the secondary balloon catheter by the inflation and deflation unit,
   c) controlled release of liquid through the irrigation lumen of the secondary balloon catheter by the flushing source unit until reaching an abort criterion, potentially combined for part of all of the perfusion period with controlled release of a different (e.g. cardioplegic) liquid through the irrigation lumen of the primary balloon catheter by the flushing source,
   d) deflating the secondary balloon by the inflation and deflation unit,
   e) controlled release of a liquid, which may or may not be identical to one of the aforementioned ones, through one of the lumens of the primary balloon catheter by the flushing source unit until reaching the abort criterion, and
   f) deflating the primary balloon by the inflation and deflation unit.

In deviation from the preferred above chronological sequence of steps a) to f), step d) can also be carried out together with the above step f), i.e. deflating of the secondary balloon by the inflation and deflation unit can be carried out during or after the controlled release of liquid through one of the lumens of the primary balloon catheter by the flushing source unit until reaching the abort criterion and together with deflating the primary balloon by the inflation and deflation unit. The main criterion of the SHIELD-EP method is the independently controlled timing, location, and composition of reperfusion, so that the distal ischemic myocardial zone recovers electrical excitability earlier than the proximal ischemic myocardial zone. After deflation of the primary and secondary balloon, the dual catheter arrangement can be removed.

In a preferred embodiment the interventional cardiology system further provides an electrical measurement unit which is connected or connectable at least with the electrode arrangements which are attached at least at the distal ends of the primary and secondary balloon catheter, each delivering measuring signals, which are used in the control unit as a basis for determining the abort criterion. In those cases in which the primary and/or secondary balloon catheter have further electrodes distributed at least in areas along the primary and/or secondary balloon catheter sleeves, these electrodes are individually connected or connectable with the electrical measurement unit for delivering measuring signals, which may be used in the control unit as a basis for determining the abort criterion also. The measuring signals obtained by means of the electrode arrangements reflect the electrical activity of the myocardium immediately adjacent to the primary and/or secondary balloon catheter, which is used to assess the state of local cardiac electrical excitability. The measured signals are compared with the electrical excitability of healthy myocardial tissue as part of a target/actual value comparison. A predefinable minimum approximation to this target value serves as an abort criterion.

### BRIEF DESCRIPTION OF THE DRAWINGS

The invention will be described below in an exemplary manner by way of embodiments with reference to the figures, without any limitation of the general inventive concept. Here:
- Fig. 1: A schematic representation of the dual catheter arrangement in combination with inflation and deflation unit, flushing source unit, electrical measurement unit and control unit, and
- Fig. 2: sequential steps for performing SHIELD-EP method using the dual catheter arrangement.

### DETAILED DESCRIPTION OF THE INVENTION

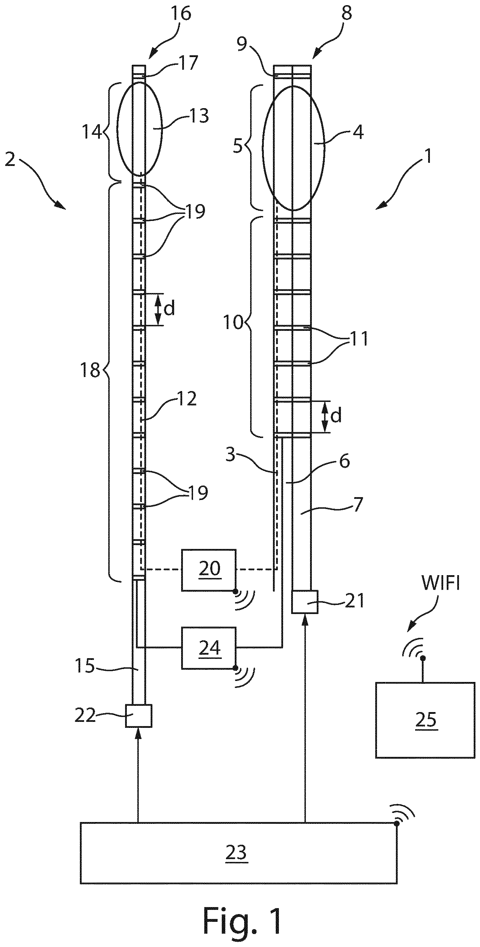

Figure 1 illustrates a system for reperfusion of an ischemic tissue region in a coronary vessel comprising a dual catheter arrangement, which consists of a primary balloon catheter 1 and a secondary balloon catheter 2.

The primary balloon catheter 1 provides a lumen 3 for inflation and deflation of a primary balloon 4 positioned at a distal region 5 of the primary balloon catheter 1, and at least two separate lumens 6, 7 both extending throughout the primary balloon catheter 1 and open at a distal end 8 of the primary balloon catheter 1. The primary balloon catheter 1 further provides at its distal end 8 a first electrode arrangement 9, preferably in the form of one or multiple ring electrodes encircling the distal end 8, and a second electrode arrangement 10, which is attached proximally to the primary balloon 4 comprising at least two electrodes 11, preferably a multitude of electrodes 11, being arranged at the outer periphery of the primary balloon catheter 1 with a distance d from each other, which isn't necessarily constant but preferably.

The secondary balloon catheter 2 has a lumen 12 for inflation and deflation of a secondary balloon 13 disposed at the distal region 14 of the secondary balloon catheter 2 and an irrigation lumen 15 opening at the distal end 16 of the secondary balloon catheter 2. The secondary balloon catheter 2 is dimensioned and formed such, that the secondary balloon catheter 2 is insertable into one of the lumens 6 of the primary balloon catheter 1 in a slidable manner, when the secondary balloon 13 is deflated. The secondary balloon catheter 2 further provides at its distal end 16 a third electrode arrangement 17, preferably in the form of one or multiple ring electrodes encircling the distal end 16, and a fourth electrode arrangement 18, which is attached proximally to the secondary balloon 13 comprising at least two electrodes 19, preferably a multitude of electrodes 19, being arranged at the outer periphery of the secondary balloon catheter 2 with a distance d from each other, which isn't necessarily constant but preferably.

The lumens 3, 12 for inflation and deflation of the primary and secondary balloon 4, 13 are fluid-tightly connected or connectable with an inflation and deflation unit 20. The irrigation lumen 15 of the secondary balloon catheter 2 and the lumen 7 which is not used for the secondary balloon catheter 2 provide at its proximal ends a connecting flange 21, 22 each for detachable fluid-tight connection to a flushing source 23. Further the first, second, third and fourth electrode arrangements 9, 10, 17, 18 on the primary and secondary balloon catheters respectively are each connected galvanically with an electrical measurement unit 24. The inflation and deflation unit 20, the flushing source unit 23, and the electrical measurement unit 24 are wired or wirelessly connected to a control unit 25 that controls the operation and activity of all units so that the operation of the units and the dual catheter assembly associated with them can be operated semi- or fully automatically. In figure 1 a radio based data exchange WIFI between the units 20, 23, 24 and 25 is illustrated.

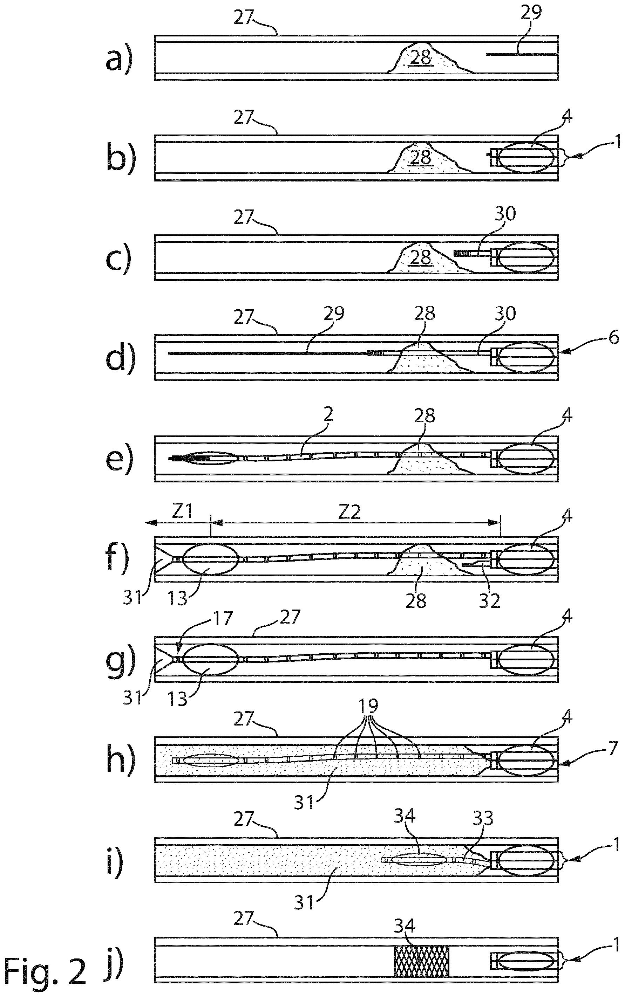

In figures 2a-j sequential steps for performing SHIELD-EP method using the dual catheter arrangement are illustrated.

Fig. 2a shows a cardiac blood vessel 27, which is occluded by a thrombus 28. Assume that the tissue area A1 to the right of the thrombus 28 is perfused, whereas the tissue area A2 to the left of the thrombus 28 is ischemic.

After identifying the position of the thrombus 28,for example by angiography, a guidewire 29 is inserted into the cardiac blood vessel 27 lumen, for example via the femoral artery, and moved next to the thrombus 28, for example under X-ray guidance.

In a next step the primary balloon catheter 1 is passed over the guidewire 29 into the blood vessel 27 and positioned in front, i.e. proximally, of the thrombus 28, see figure 2b. For this purpose, the lumen 6 of the primary balloon catheter 1 passes over guide wire 29. The primary balloon 4 is then inflated, ensuring the blood vessel 27 remains blocked and unharmed and the primary catheter remains firmly positioned.

After inflating the primary balloon 4 a thrombus crossing catheter 30 is then introduced in the case of a total occlusion of the blood vessel 27 by the thrombus 28, see figure 2c. In case of an incomplete occlusion this step could be skipped.

The thrombus crossing catheter 30 passed through a lumen 6 of the primary balloon catheter 1 over the guidewire 29, see figure 2d. Once the thrombus 28 is crossed the guidewire 29 is then further advanced distally while the thrombus crossing catheter 30 is removed proximally and in its place the secondary balloon catheter 2 is introduced into the lumen 6 of the primary balloon catheter 1, see figure 2e.

The secondary balloon 13 then is inflated in a working position, in which the secondary balloon 13 is within the ischemic area, distally of the occluded vessel area, and further divides this ischemic area in a distal ischemic myocardial zone Z1, distally to the secondary balloon 13, and a proximal ischemic myocardial zone Z2, which is between the primary and secondary balloon 4, 13, see figure 2f.

Once in the working position perfusion of the distal ischemic zone Z1 is started, by releasing of perfusion solution 31 through the irrigation lumen of the secondary balloon catheter 2. At the same time an aspiration catheter 32 may be used in the other lumen 6 of the primary balloon catheter 1 to remove the thrombus 28, see figure 2f again.

The aspiration catheter is removed upon removal of the thrombus 28, while perfusion is continued until the distal zone Z1 is once again excitable as measured using the distal electrode 17 of the secondary balloon catheter 2, see figure 2g.

The secondary balloon 13 is then deflated, the secondary balloon catheter 2 can remain in place and perfusion of the proximal myocardial zone Z2 is started, see figure 2h, by a controlled release of perfusion solution through the lumen 7 of the primary balloon catheter 1 until the proximal zone Z2 is once again excitable as measured using the electrodes 19 of the secondary balloon catheter 2 and the distal electrode arrangement 9 of the primary balloon catheter 1, see figure 2h. The perfusion solution released through the lumen 7 of the primary balloon catheter 1 also reaches the distal area Z1, since the secondary balloon is deflated and thus no longer represents a fluid barrier. During or after perfusion of the proximal myocardial zone Z2 a stent catheter 33 having a stent 34 can be introduced through a lumen of the primary balloon catheter 1, see figure 2i.

After the stent 34 is placed in the region of the removed thrombus, the remaining primary balloon 4 is deflated and subsequently all catheters are removed, see figure 2j.

The proposed stepwise reperfusion technique SHIELD-EP can be characterized by the following steps:
A method of spatially and temporally controlled reperfusion of two distinct zones an ischemic tissue region with defined solutions (type, flow rate and pressure, temperature, oxygenation, etc.) in a coronary vessel caused by an occlusion along a blood-carrying vessel, comprising the steps of:
- locating the occlusion and placing a primary balloon proximal to the occlusion within the vessel such that tissue region adjacent to the vessel is perfused with blood proximally to the primary balloon and is ischemic distally,
- placing a secondary balloon distal to the occlusion within the vessel in the ischemic tissue region, which together with the first balloon limit an interstitial space, further referred as proximal myocardial zone Z2, and further limits a distal myocardial zone Z1 distal to the secondary balloon,
- perfusing the distal myocardial zone Z1 with a perfusion solution, while proximal myocardial zone Z2 remains untreated and ischemic until reaching an abort criterion or subjected to separately controlled perfusion with a suitable solution that keep proximal tissue non-excitable,
- perfusing at least the proximal myocardial zone Z2 with a perfusion solution until reaching an abort criterion
- removing the primary and secondary balloon from the vessel.

The time gap between both perfusing steps can be specified individually. The distance between the primary and secondary balloons can be specified individually as well. The flow rates and perfusion pressures for the perfusion solution in each perfusion line is controlled independently. Also it is possible release a selected liquid of at least two different liquids through each of the lumens of both catheters.

Further it is of advantage to remove the occlusion after placing at least the primary balloon, preferably after placing both, the primary and the secondary balloon. After removing all the balloons it may be of advantage to implement a stent at the location of the removed occlusion for avoiding any local later constriction.

The invention is defined by the following claims.

### List of reference signs

- 1: primary balloon catheter
- 2: secondary balloon catheter
- 3: lumen for inflation and deflation
- 4: primary balloon
- 5: distal region of primary balloon catheter
- 6: lumen of primary balloon catheter
- 7: lumen of primary balloon catheter
- 8: distal end of primary balloon catheter
- 9: first electrode arrangement of primary balloon catheter
- 10: second electrode arrangement of primary balloon catheter
- 11: electrodes of primary balloon catheter
- 12: lumen
- 13: secondary balloon
- 14: distal region of secondary balloon catheter
- 15: irrigation lumen
- 16: distal end of secondary balloon catheter
- 17: third electrode arrangement of secondary balloon catheter
- 18: fourth electrode arrangement of secondary balloon catheter
- 19: electrodes of secondary balloon catheter
- 20: inflation and deflation unit
- 21: connecting flange
- 22: connecting flange
- 23: flushing source unit
- 24: electrical measurement unit
- 25: control unit
- 26: thrombus
- 27: cardiac blood vessel
- 28: thrombus
- 29: guidewire
- 30: thrombus crossing catheter
- 31: perfusion solution
- 32: aspiration catheter
- 33: stent catheter
- 34: stent
- Z1: distal ischemic zone
- Z2: proximal ischemic myocardial zone

## Claims

1. A dual catheter arrangement for reperfusion of an ischemic tissue region via a coronary vessel, comprising a primary balloon catheter (1) having a lumen (3) for inflation and deflation of a primary balloon (4) disposed at a distal region (5) of the primary balloon catheter (1) and a lumen (6) extending throughout the primary balloon catheter (1) and open at a distal end (8) of the primary balloon catheter (1), and a secondary balloon catheter (2) having a lumen (12) for inflation and deflation of a secondary balloon (13) disposed at a distal region (14) of the secondary balloon catheter (2), which is arranged slidable within the lumen (6) of the primary balloon catheter (1) to achieve a working position in which the secondary balloon catheter (2) continues completely through the lumen (6) of the primary balloon catheter (1) and extends distally beyond the primary balloon catheter (1), **characterised in that** the primary balloon catheter (1) has at least a further separate lumen (7) extending throughout the primary balloon catheter (1) having a connecting flange (21) proximally for detachable, fluid-tight connection to a flushing source unit (23) and open at the distal end (8) of the primary balloon catheter (1), and the secondary balloon catheter (2) has an irrigation lumen (15) having a connecting flange (22) proximally for detachable, fluid-tight connection to a flushing source unit (23) and an opening at a distal end (16) of the secondary balloon catheter (2).

2. The dual catheter arrangement according to claim 1,
wherein a first electrode arrangement (9) is attached at least at the distal end (8) of the primary balloon catheter (1) being designed to detect an electrical activity of a tissue surrounding the distal end (8) of the first balloon catheter (1).

3. The dual catheter arrangement according to claim 1 or 2,
wherein a second electrode arrangement (10) is attached proximally to the primary balloon (4) at least in areas along the primary balloon catheter (1), being designed to detect an electrical activity of a tissue surrounding the primary balloon catheter (1).

4. The dual catheter arrangement according to one of the claims 1 to 3,
wherein a third electrode arrangement (17) is attached at the distal end (16) of the secondary balloon catheter (2), which is designed to detect an electrical activity of a tissue surrounding the distal end (16) of the secondary balloon catheter (2).

5. The dual catheter arrangement according to one of the claims 1 to 4,
wherein a fourth electrode arrangement (18) is attached proximally to the secondary balloon (13) at least in areas along the secondary balloon catheter (2), being designed to detect an electrical activity of a tissue surrounding the second balloon catheter (2).

6. The dual catheter arrangement according to claim 3 and 5,
wherein the second electrode arrangement (10) provides at least two electrodes (11) being arranged at a distance from each other along an outer periphery of the primary balloon catheter (1) and
wherein the fourth electrode arrangement (18) provides at least two electrodes (19) being arranged at a distance from each other along an outer periphery of the secondary balloon catheter (2).

7. The dual catheter arrangement according to one of the claims 1 to 6, wherein the secondary balloon catheter (2) provides a catheter length which is greater than a length of the primary balloon catheter (1), so that, in the working position, the secondary balloon catheter (2) towers the primary balloon catheter (1) distalwards by a variable adjustable length of up to 10 cm.

8. System for reperfusion of an ischemic tissue region via a coronary vessel comprising the dual catheter arrangement of one of the claims 1 to 7, further comprising
- an inflation and deflation unit (20) connected fluid-tightly with the lumen (3, 19) for inflation and deflation of the primary and secondary balloon (4, 13),
- a flushing source unit (23) connected fluid-tightly with at least one of the at least two lumens (6, 7) of the primary balloon catheter (1) and with the irrigation lumen (15) of the secondary balloon catheter (2), and
- a control unit (25) for controlling the inflation and deflation unit (20) and the flushing source unit (23) in the following manner:
- inflating the primary balloon (4) by the inflation and deflation unit (20),
- after placing the secondary balloon catheter (2) in the working position, inflating the secondary balloon (13) by the inflation and deflation unit (20),
- controlled release of liquid through the irrigation lumen (15) of the secondary balloon catheter (2) by the flushing source unit (23) until reaching an abort criterion,
- deflating the secondary balloon (13) by the inflation and deflation unit (20),
- controlled release of liquid through one of the lumens (6, 7) of the primary balloon catheter (1) by the flushing source unit (23) until reaching the abort criterion, and
- deflating the primary balloon (4) by the inflation and deflation unit (20).

9. System according to claim 8 with the dual catheter arrangement according to claims 2 and 4,
wherein an electrical measurement unit (24) is connected at least with the first and third electrode arrangement (9, 17) delivering measuring signals, which are used in the control unit (25) as a basis for determining the abort criterion.

10. System according to claim with the dual catheter arrangement according to claims 3 and 5,
wherein the electrical measurement unit (24) is connected at least with the second and fourth electrode arrangement (10, 18) delivering measuring signals, which are used in the control unit (25) as a basis for determining the abort criterion.

11. System according to one of the claims 8 to 10, wherein the control unit (25) controls the flushing source unit (23) such that at least one of the at least two lumens (6, 7) of the primary balloon catheter (1) and the irrigation lumen (15) of the secondary balloon catheter (2) can be flushed independently with control over time, pressure, flow, temperature and /or type of liquid.

12. System according to one of the claims 8 to 11, wherein the control unit (25) starts the controlled release of liquid through one of the lumens (6, 7) of the primary balloon catheter (1) by the flushing source unit (23) timely before or during deflating the secondary balloon (13) by the inflation and deflation unit (20).

## Patentansprüche

1. Doppelkatheteranordnung zur Reperfusion eines ischämischen Gewebebereichs über ein Herzkranzgefäß, umfassend einen primären Ballonkatheter (1), der ein Lumen (3) zum Aufblasen und Ablassen eines primären Ballons (4), der an einem distalen Bereich (5) des primären Ballonkatheters (1) angeordnet ist, und ein Lumen (6), das sich durch den gesamten primären Ballonkatheter (1) erstreckt und an einem distalen Ende (8) des primären Ballonkatheters (1) offen mündet, aufweist, und einen sekundären Ballonkatheter (2), der ein Lumen (12) zum Aufblasen und Ablassen eines sekundären Ballons (13), der an einem distalen Bereich (14) des sekundären Ballonkatheters (2) angeordnet ist, aufweist, der verschiebbar innerhalb des Lumens (6) des primären Ballonkatheters (1) angeordnet ist, um eine Arbeitsposition zu erreichen, in welcher sich der sekundäre Ballonkatheter (2) vollständig durch das Lumen (6) des primären Ballonkatheters (1) erstreckt und sich in Distalrichtung über den primären Ballonkatheter (1) hinaus erstreckt,
**dadurch gekennzeichnet, dass**
der primäre Ballonkatheter (1) zumindest ein weiteres gesondertes Lumen (7) aufweist, das sich durch den gesamten primären Ballonkatheter (1) erstreckt, wobei es einen Verbindungsflansch (21) in Proximalrichtung für eine abtrennbare, fluiddichte Verbindung mit einer Spülquelleneinheit (23) und offen an dem distalen Ende (8) des primären Ballonkatheters (1) mündet, und der sekundäre Ballonkatheter (2) ein Spüllumen (15) aufweist, das einen Verbindungsflansch (22) in Proximalrichtung für eine abtrennbare, fluiddichte Verbindung mit einer Spülquelleneinheit (23) und eine Öffnung an einem distalen Ende (16) des sekundären Ballonkatheters (2) aufweist.

2. Doppelkatheteranordnung nach Anspruch 1,
wobei eine erste Elektrodenanordnung (9) zumindest an dem distalen Ende (8) des primären Ballonkatheters (1) angeordnet ist, wobei sie dafür ausgelegt ist, eine elektrische Aktivität eines Gewebes, welches das distale Ende (8) des primären Ballonkatheters (1) umgibt, zu erfassen.

3. Doppelkatheteranordnung nach Anspruch 1 oder 2,
wobei eine zweite Elektrodenanordnung (10) proximal zu dem primären Ballon (4) zumindest in Bereichen entlang des primären Ballonkatheters (1) befestigt ist, wobei sie dafür ausgelegt ist, eine elektrische Aktivität eines Gewebes, welches den primären Ballonkatheter (1) umgibt, zu erfassen.

4. Doppelkatheteranordnung nach einem der Ansprüche 1 bis 3,
wobei eine dritte Elektrodenanordnung (17) an dem distalen Ende (16) des sekundären Ballonkatheters (2) angeordnet ist, die dafür ausgelegt ist, eine elektrische Aktivität eines Gewebes, welches das distale Ende (16) des sekundären Ballonkatheters (2) umgibt, zu erfassen.

5. Doppelkatheteranordnung nach einem der Ansprüche 1 bis 4,
wobei eine vierte Elektrodenanordnung (18) proximal zu dem sekundären Ballon (13) zumindest in Bereichen entlang des sekundären Ballonkatheters (2) befestigt ist, wobei sie dafür ausgelegt ist, eine elektrische Aktivität eines Gewebes, welches den sekundären Ballonkatheter (2) umgibt, zu erfassen.

6. Doppelkatheteranordnung nach Anspruch 3 und 5,
wobei die zweite Elektrodenanordnung (10) mindestens zwei Elektroden (11) bereitstellt, die in einer Entfernung voneinander entlang eines äußeren Umfangs des primären Ballonkatheters (1) angeordnet sind, und
wobei die vierte Elektrodenanordnung (18) mindestens zwei Elektroden (19) bereitstellt, die in einer Entfernung voneinander entlang eines äußeren Umfangs des sekundären Ballonkatheters (2) angeordnet sind.

7. Doppelkatheteranordnung nach einem der Ansprüche 1 bis 6,
wobei der sekundäre Ballonkatheter (2) eine Katheterlänge bereitstellt, die größer ist als eine Länge des primären Ballonkatheters (1), so dass, in der Arbeitsposition, der sekundäre Ballonkatheter (2) den primären Ballonkatheter (1) in Distalrichtung um eine veränderliche einstellbare Länge von bis zu 10 cm überragt.

8. System zur Reperfusion eines ischämischen Gewebebereichs über ein Herzkranzgefäß, wobei das System die Doppelkatheteranordnung nach einem der Ansprüche 1 bis 7 umfasst, wobei es ferner Folgendes umfasst:
- eine Aufblas- und Ablasseinheit (20), die fluiddicht mit dem Lumen (3, 19) verbunden ist, zum Aufblasen und Ablassen des primären und des sekundären Ballons (4, 13),
- eine Spülquelleneinheit (23), die fluiddicht mit mindestens einem von den mindestens zwei Lumina (6, 7) des primären Ballonkatheters (1) und mit dem Spüllumen (15) des sekundären Ballonkatheters (2) verbunden ist,
- eine Steuereinheit (25) zum Steuern der Aufblas- und Ablasseinheit (20) und der Spülquelleneinheit (23) auf die folgende Weise:
- Aufblasen des primären Ballons (4) durch die Aufblas- und Ablasseinheit (20),
- nach einem Platzieren des sekundären Ballonkatheters (2) in der Arbeitsposition Aufblasen des sekundären Ballons (13) durch die Aufblas- und Ablasseinheit (20),
- geregelte Freisetzung von Flüssigkeit durch das Spüllumen (15) des sekundären Ballonkatheters (2) durch die Spülquelleneinheit (23) bis zum Erreichen eines Abbruchkriteriums,
- Ablassen des sekundären Ballons (13) durch die Aufblas- und Ablasseinheit (20),
- geregelte Freisetzung von Flüssigkeit durch eines der Lumina (6, 7) des primären Ballonkatheters (1) durch die Spülquelleneinheit (23) bis zum Erreichen des Abbruchkriteriums und
- Ablassen des primären Ballons (4) durch die Aufblas- und Ablasseinheit (20).

9. System nach Anspruch 8 mit der Doppelkatheteranordnung nach Anspruch 2 und 4,
wobei eine elektrische Messeinheit (24) zumindest mit der ersten und der dritten Elektrodenanordnung (9, 17) verbunden ist, wobei sie Messsignale liefert, die in der Steuereinheit (25) als eine Grundlage zum Festlegen des Abbruchkriteriums verwendet werden.

10. System nach Anspruch 9 mit der Doppelkatheteranordnung nach Anspruch 3 und 5,
wobei die elektrische Messeinheit (24) zumindest mit der zweiten und der vierten Elektrodenanordnung (10, 18) verbunden ist, wobei sie Messsignale liefert, die in der Steuereinheit (25) als eine Grundlage zum Festlegen des Abbruchkriteriums verwendet werden.

11. System nach einem der Ansprüche 8 bis 10,
wobei die Steuereinheit (25) die Spülquelleneinheit (23) derart steuert, dass zumindest eines von den mindestens zwei Lumina (6, 7) des primären Ballonkatheters (1) und dem Spüllumen (15) des sekundären Ballonkatheters (2) unabhängig mit Kontrolle über Zeit, Druck, Durchflussmenge, Temperatur und/oder Art der Flüssigkeit gespült werden kann.

12. System nach einem der Ansprüche 8 bis 11,
wobei die Steuereinheit (25) die geregelte Freisetzung von Flüssigkeit durch eines der Lumina (6, 7) des primären Ballonkatheters (1) durch die Spülquelleneinheit (23) rechtzeitig vor dem Auflasen des sekundären Ballons (13) durch die Aufblas- und Ablasseinheit (20) oder währenddessen startet.

## Revendications

1. Agencement de cathéter double pour la re-perfusion d'une région de tissus ischémique par l'intermédiaire d'un vaisseau coronaire, comprenant un cathéter à ballonnet principal (1) ayant une lumière (3) pour gonfler et dégonfler un ballonnet principal (4) disposé sur une région distale (5) du cathéter à ballonnet principal (1) et une lumière (6) s'étendant à travers le cathéter à ballonnet principal (1) et ouverte à une extrémité distale (8) du cathéter à ballonnet principal (1), et un cathéter à ballonnet secondaire (2) ayant une lumière (12) pour gonfler et dégonfler un ballonnet secondaire (13) disposé à une région distale (14) du cathéter à ballonnet secondaire (2) qui est agencé en pouvant coulisser à l'intérieur de la lumière (6) du cathéter à ballonnet principal (1) pour atteindre une position de travail dans laquelle le cathéter à ballonnet secondaire (2) continue complètement à travers la lumière (6) du cathéter à ballonnet principal (1) et s'étend de manière distale au-delà du cathéter à ballonnet principal (1),
**caractérisé en ce que** le cathéter à ballonnet principal (1) présente au moins une lumière supplémentaire séparée (7) s'étendant à travers le cathéter à ballonnet principal (1) ayant une bride de connexion (21) orientée de façon proximale pour une connexion séparable étanche aux fluides à une unité de source de rinçage (23) et ouverte à l'extrémité distale (8) du cathéter à ballonnet principal (1), et le cathéter à ballonnet secondaire (2) présente une lumière d'irrigation (15) ayant une bride de connexion (22) orientée de façon proximale pour une connexion séparable étanche aux fluides à une unité de source de rinçage (23) et une ouverture à une extrémité distale (16) du cathéter à ballonnet secondaire (2).

2. Agencement de cathéter double selon la revendication 1, dans lequel un premier agencement d'électrodes (9) est fixé au moins sur l'extrémité distale (8) du cathéter à ballonnet principal (1) étant conçu pour détecter une activité électrique d'un tissu entourant l'extrémité distale (8) du cathéter à ballonnet principal (1).

3. Agencement de cathéter double selon la revendication 1 ou 2, dans lequel un deuxième agencement d'électrodes (10) est fixé de manière proximale au ballonnet principal (4) au moins dans des zones le long du cathéter à ballonnet principal (1), étant conçu pour détecter une activité électrique d'un tissu entourant le cathéter à ballonnet principal (1).

4. Agencement de cathéter double selon l'une des revendications 1 à 3, dans lequel un troisième agencement d'électrodes (17) est fixé à l'extrémité distale (16) du cathéter à ballonnet secondaire (2), qui est conçu pour détecter une activité électrique d'un tissu entourant l'extrémité distale (16) du cathéter à ballonnet secondaire (2).

5. Agencement de cathéter double selon l'une des revendications 1 à 4, dans lequel un quatrième agencement d'électrodes (18) est fixé de manière proximale au ballonnet secondaire (13) au moins dans des zones le long du cathéter à ballonnet secondaire (2), étant conçu pour détecter une activité électrique d'un tissu entourant le cathéter à ballonnet secondaire (2).

6. Agencement de cathéter double selon la revendication 3 et 5, dans lequel le deuxième agencement d'électrodes (10) fournit au moins deux électrodes (11) étant agencées à distance l'une de l'autre le long d'une périphérie extérieure du cathéter à ballonnet principal (1) et
dans lequel le quatrième agencement d'électrodes (18) fournit au moins deux électrodes (19) agencées à distance l'une de l'autre le long d'une périphérie extérieure du cathéter à ballonnet secondaire (2).

7. Agencement de cathéter double selon l'une des revendications 1 à 6, dans lequel le cathéter à ballonnet secondaire (2) fournit une longueur de cathéter qui est supérieure à une longueur du cathéter à ballonnet principal (1) de façon à ce que dans la position de travail, le cathéter à ballonnet secondaire (2) domine le cathéter à ballonnet principal (1) vers le côté distal d'une longueur variable ajustable jusqu'à 10 cm.

8. Système de re-perfusion d'une région de tissus ischémique par l'intermédiaire d'un vaisseau coronaire, comprenant l'agencement de cathéter double selon l'une des revendications 1 à 7, comprenant en outre :
- une unité de gonflage et de dégonflage (20) connectée en étanchéité aux fluides à la lumière (3, 19) pour le gonflage et le dégonflage des ballonnets principal et secondaire (4, 13),
- une unité de source de rinçage (23) connectée en étanchéité fluidique à au moins une des au moins deux lumières (6, 7) du cathéter à ballonnet principal (1) et à la lumière d'irrigation (15) du cathéter à ballonnet secondaire (2), et
- une unité de commande (25) pour commander l'unité de gonflage et de dégonflage (20) et l'unité de source de rinçage (23) de la manière suivante :
- gonflage du ballonnet principal (4) par l'unité de gonflage et de dégonflage (20),
- après le placement du cathéter à ballonnet secondaire (2) dans la position de travail, gonflage du ballonnet secondaire (13) par l'unité de gonflage et de dégonflage (20),
- libération commandée de liquide à travers la lumière d'irrigation (15) du cathéter à ballonnet secondaire (2) par l'unité de source de rinçage (23) jusqu'à obtenir un critère d'interruption,
- dégonflage du ballonnet secondaire (13) par l'unité de gonflage et de dégonflage (20),
- libération commandée de liquide à travers une des lumières (6, 7) du cathéter à ballonnet principal (1) par l'unité de source de rinçage (23) jusqu'à atteindre le critère d'abandon, et
- dégonflage du ballonnet primaire (4) par l'unité de gonflage et de dégonflage (20).

9. Système selon la revendication 8, comprenant l'agencement de cathéter double selon les revendications 2 et 4,
dans lequel une unité de mesure électrique (24) est connectée au moins au premier et au troisième agencement d'électrodes (9, 17) fournissant des signaux de mesure qui sont utilisés dans l'unité de commande (25) en tant que base pour déterminer le critère d'abandon.

10. Système selon la revendication 9, comprenant l'agencement de cathéter double selon les revendications 3 et 5,
dans lequel l'unité de mesure électrique (24) est connectée au moins au deuxième et quatrième agencement d'électrodes (10, 18) fournissant des signaux de mesure, qui sont utilisés dans l'unité de commande (25) en tant que base pour déterminer le critère d'abandon.

11. Système selon l'une des revendications 8 à 10, dans lequel l'unité de commande (25) commande l'unité de source de rinçage (23) de telle façon qu'au moins une des deux lumières (6, 7) du cathéter à ballonnet principal (1) et la lumière d'irrigation (15) du cathéter à ballonnet secondaire (2) puissent être rincées indépendamment avec une commande sur la durée, la pression, le débit, la température et/ou le type de liquide.

12. Système selon l'une des revendications 8 à 11, dans lequel l'unité de commande (25) démarre la libération commandée de liquide à travers l'une des lumières (6, 7) du cathéter à ballonnet principal (1) par l'unité de source de rinçage (23) à temps avant ou pendant le dégonflage du ballonnet secondaire (13) par l'unité de gonflage et dégonflage (20).
